# EUROPEAN PATENT SPECIFICATION

(11) **EP 0 220 236 B1**
(45) Date of publication and mention of the grant of the patent: **02.03.1994**
(21) Application number: 86902704.5
(22) Date of filing: 07.04.1986
(51) Int. Cl.: A61M 5/00, A61M 25/00

(54) **METHOD FOR THE PREVENTION OF RESTENOSIS**
VORBEUGEN VON RESTENOSE
METHODE DE PREVENTION DE LA RESTENOSIS

(30) Priority: 09.04.1985 US 721386; 24.01.1986 US 822014
(43) Date of publication of application: 06.05.1987
(73) Proprietor: WOLINSKY, Harvey, New York NY 10024 (US)
(72) Inventor: WOLINSKY, Harvey, New York NY 10024 (US)
(74) Representative: Weinhold, Peter, Dr.
(86) International application number: US8600747
(87) International publication number: WO8605990

(56) References cited:
- US-A- 4 299 226
- US-A- 4 423 725
- US-A- 4 445 892
- US-A- 4 573 966
- Circulation Research, Guyton et al.,Vol.46, Nr. 5, pages 625-633, 1980

## Description

Recently an alternative approach to coronary bypass surgery has been developed. In this non-operative procedure for the improvement of blood flow in patients with coronary artery disease, a catheter with an inflatable balloon at the distal end is inserted into the femoral artery or by brachial cutdown, and is positioned by fluoroscopic control at the appropriate coronary ostium. The process is known as percutaneous transluminal coronary angioplasty (PTCA).

The balloon at the distal end of the catheter has a predetermined maximum diameter. It is filled with a radio opaque dye to permit visualization. Alternatively, the balloon itself may be radio opaque. Then the balloon is positioned in the stenosis and inflated at pressures of from 2 to 11 atmospheres for from 15 to 60 seconds and then deflated. The inflation cycle may be repeated several times to achieve satisfactory results. Normally the luminal diameter of the stenotic vessel increases by at least 20% as a result of the treatment.

Angioplasty is not limited to the cardiac vasculature. It has been employed for treatment of single, large atherosclerotic lesions of the renal, iliac and even vertebral arteries. The effect of the expanded balloon is to literally blow open the stenotic zone. Disruption of the wall is marked, including fracture of the calcium in the lesion, tearing of the plaque itself and extravasation of plaque lipid and gruel into the adjacent vessel wall.

The clinical results of angioplasty include endothelial denudation, vascular wall damage, and rupture of the tunica intima vasorum. These injuries have been found to result in many cases in unregulated proliferation of the arterial smooth muscle cells (SMC) with a resulting restenosis. A recent study by Levine et al.(The American Journal of Cardiology, Volume 55, pages 673 to 676, March 1985) has shown that restenosis may be expected to occur in as many as 40% of patients that have undergone angioplasty. Often the only practical treatment for restenosis is to repeat the treatment. This may cause further damage to the cell wall and the need for subsequent repetition of the angioplasty procedure.

Heparin is a mucopolysaccharide composed of amino sugar and uronic acid residues which is obtained from beef, porcine, sheep, whale and other mammalian tissue by extraction with a solution of postassium acetate, alkaline ammonium sulfate and the like. Commercial heparin preparations are now widely available from a number of pharmaceutical companies. Heparin preparations are clinically utilized principally as anticoagulants.

US-A-4 423 725 describes a multiple surgical cuff for introduction into a body passage such as a trachea, bladder and urethra opening or artery comprising a tubular base member encircled by proximal distal and middle double cuff members. It is stated that one advantage of said surgical cuff is that drugs such as anticoagulant drugs (like heparin) can be administered thereby under pressure,forcing such drugs to penetrate deep into the tissue or gland ducts.

US-A-4 299 226 describes a coronary dilation method wherein a guiding catheter having at least one side arm is continuously flushed with heparinized saline during the dilation process.

Recently it has become known that in addition to its anticoagulant activities, heparin is a powerful inhibitor of arterial smooth muscle proliferation. See, for example, Guyton et al. Circulation Research Volume 46, Number 5 pages 625 to 633, 1980 and Hoover et al. Circulation Research Volume 47, Number 4, pages 578 to 583, 1980.

According to the present invention it has been found that for the efficient prevention of restenosis following angioplasty heparin must be administered at the site of angioplasty at a pressure of from 200 to 1000 mm Hg for 5 to 60 seconds.

Accordingly the present invention is directed to the use of heparin for the manufacture of a medicament for the prevention of restenosis following angioplasty by administration at the site of angioplasty in the above manner.

WO 83/03356 describes catheters which can be used to insert a solubilizing agent into an artery to dissolve plaque, thereby relieving arterial constrictions.

In the drawing:
Figure 1 is a schematic longitudinal sectional view of a catheter element which may be employed in connection with this invention at the distal end of a main catheter body.
Figure 2 is a cross section taken along the line 2-2 of Figure 1.
Figure 3 is a view of the catheter element of Figure 1 operatively positioned within a stenotic artery.

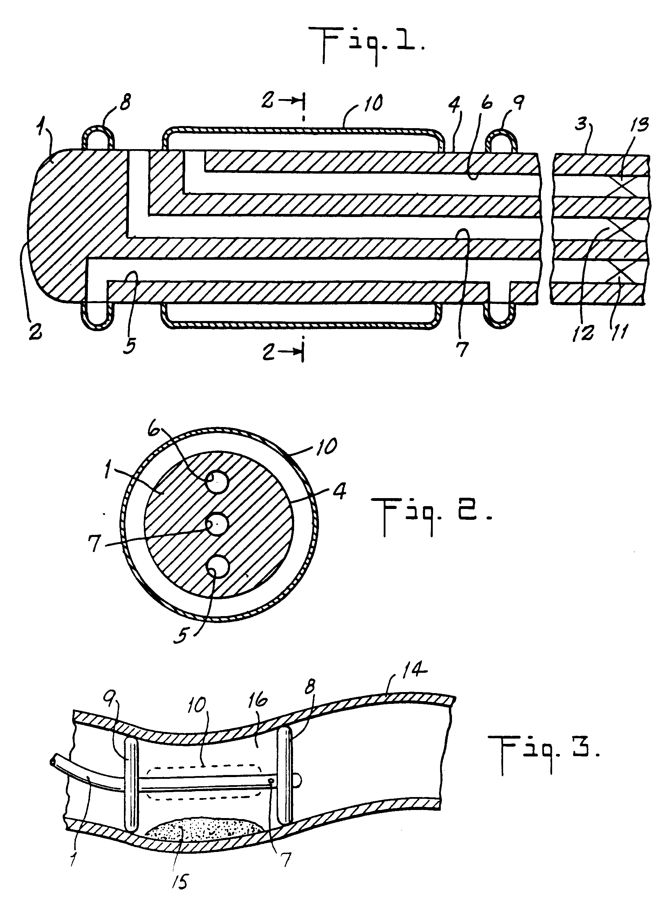

Figures 1 and 2 illustrate the solubilizing fluid delivery, balloon carrying element of a catheter useful for the delivery of heparin at the site of angioplasty. In the embodiment illustrated it comprises a main catheter body generally designated as 1 with a distal end 2 and a proximate end 3 formed with a main catheter body wall 4. The main catheter body 1 is formed with three conduits; a ring balloon expansion conduit 5, a central balloon expansion conduit 6 and a fluid delivery conduit 7. The catheter body 1 carries two ring balloons 8 and 9 at either end, and an optional central balloon 10 disposed intermediate the spaced balloons. It also carries a third conduit 7 which exits through the catheter body. Conduits 5, 6 and 7 are fitted with appropriate valves 11, 12 and 13.

Catheters of the class described are useful for delivering heparin to the site of the angioplasty and depositing it in and about the site of the vascular wall damage to retard SMC growth.

The term 'heparin' as used herein refers to any of a variety of heparin products which inhibit SMC proliferation. Heparin from various sources is known to be heterogeneous. There are both anticoagulant and non-anticoagulant fractions. Each has varying degrees of N- and O- sulfation and acetylation. Fractions with anticoagulant activity may contain as many as 20 saccharide moieties. It has been found that both anticoagulant and non-anticoagulant fractions manifest inhibition of SMC proliferation, and that heparin fractions or derivatives containing at least six saccharide monomers have this activity. Fractions and derivatives with varying degrees of sulfation manifest varying abilities to inhibit SMC proliferation. The active materials are described in detail in the Circulation Research publications cited above. All such fractions and derivatives are useful in the practice of this invention and are included within the term heparin.

The operation of the catheter to form a chamber within the artery is schematically illustrated in Figure 3.

In Figure 3, 14 is the arterial wall of an artery constricted due to the presence of plaque body 15. The figure shows the main catheter body 1 held in place by the inflation of spaced balloons 8 and 9. The inflation of the balloons forms a chamber 16 in the artery and, as shown, surrounding the plaque. The catheter 1 is shown with the central balloon 10 in the deflated configuration. It also shows the delivery end of the third conduit 7.

The two balloon catheter illustrated in the figures may be employed following conventional angioplasty which removes at least a portion of the plaque. The angioplasty catheter is removed and the catheter 1 is inserted. The catheter 1 is guided by standard procedures which may include the use of a flexible probe, a guide wire and/or a fluoroscope to a position overlaying the original site of the plaque body 15 preferably, but not necessarily, in the position shown in Figure 3 with the distal end balloon 8 just beyond the distal end of the original site and proximate end balloon 9 just ahead of the proximate end of the site. When the balloons 8 and 9 are inflated by forcing fluid such as isotonic saline through valve 11 and conduit 5, the catheter is held in place by the pressure of the balloons and a chamber 16 is formed surrounding the site. The closing of valve 11 will maintain the pressure in the conduit 5 and balloons 8 and 9 so that the catheter is held in place. The position of the catheter can be checked fluoroscopically or by passing a small amount of solubilizing liquid containing a dye into the chamber. If the position is not satisfactory the pressure can be released sufficiently to slightly deflate ring balloons 8 and 9, the catheter moved in the appropriate direction, and the balloons reinflated.

Once the catheter is in place, the heparin is forced under pressure through the conduit 7 and the chamber 16 on and into the adjacent surfaces. Pressures of 200 to 1000 mm Hg are sufficient for this purpose. The preferred range is 300 to 1000 mm Hg. The pressure at which the fluid is forced into the chamber may be generated by a pump upstream of valve 12. After the heparin is injected, the catheter is held in place for 5 to 60 seconds to hold the heparin in the chamber and provide time for it to stick to and penetrate the depths of the adjacent arterial tissue defined by the chamber in high concentration and not be prematurely washed away or diluted with the flowing blood. Balloons 8 and 9 are deflated, and the catheter removed.

Because of the large number of functional groups present, heparin is a highly charged molecule. When forced through the chamber 16 it enters the damaged wall and readily interacts with the surfaces of the various cells within the injured wall, as well as with the connective tissue between the cells. In effect it "sticks to" the injured site and inhibits, but does not completely stop the multiplying of SMC. Because heparin is "sticky" it will stay in an effective position until the injury is healed.

The general process by which the injured artery repairs itself involves the bathing of the injured area with platelets and other cell growth promoters in the blood. The cells within the injured area of arterial wall continue to divide and multiply to generate new cellular tissue and repair the wound. When the growth reaches the appropriate level, the body's feedback mechanism signals the growth to stop. Restenosis occurs when the feed back mechanism is not functioning properly and the SMC continues to multiply in an uncontrolled manner in the damaged angioplasty site. The presence of the heparin appears in some manner to control the multiplication of the SMC cells so that they continue to multiply, but in a controlled manner until the regular control mechanism of the body takes over. The heparin affects only the deeper SMC cells and does not affect the surface endothelial cells.

An alternative procedure to the use of the two balloon catheter as described above is to use the three balloon catheter. In this method the catheter is inserted and placed over the plaque using the procedure described. The first step is to inflate the middle balloon 10 to rupture the plaque. The balloon is deflated; after restoration of blood flow for a brief period, expansion of balloons 8 and 9 creates a chamber around the angioplasty site. The heparin is then administered as described above. The chamber is held in place for 5 to 60 seconds so that the heparin can stick to and enter the adjacent surfaces, the balloons are deflated and then the catheter is removed.

The catheter body can be prepared from any of a number of readily available, non-toxic, flexible polymers including, for example, polyolefins, such as polyethylene or polypropylene, and polyvinyl halides, such as polyvinyl chloride or polyvinylidene chloride. The balloon can be fabricated from similar materials manufactured so as to be expansible under pressure and with sufficient elasticity to collapse when the pressure is released and negative pressure applied. The dimensions of the balloons will be such that they will reach the desired diameter at a pressure of from about 75 to 150mm Hg and hold the dimensions even if the pressure is increased to as high as 5 or more atmospheres.

The absolute dimensions selected for the balloons will depend upon the diameter of the arteries involved. For example, the ring balloons may be from 2 to 5 mm in length and their expanded diameters will be approximately the same. The central balloon will be of the same diameter range as the end balloons, but the length will be from about 10 to 50 mm.

## Claims

1. Use of heparin for the manufacture of a medicament for the prevention of restenosis following angioplasty by administration at the site of angioplasty at a pressure of from 200 to 1000 mmHg and for 5 to 60 seconds.

## Patentansprüche

1. Verwendung von Heparin für die Herstellung eines Arzneimittels zur Vorbeugung gegen Restenose als Folge von Gefäßplastik durch Administrierung am Ort der Gefäßplastik bei einem Druck von 200 bis 1000 mm Hg und 5 bis 60 Sekunden lang.

## Revendications

1. Utilisation d'héparine pour la fabrication d'un médicament pour la prévention de la resténose qui suit une angioplastie par administration au site de l'angioplastie, à une pression de 200 à 1000 mm Hg et durant 5 à 60 secondes.
